# EUROPEAN PATENT APPLICATION

(11) **EP 4 548 941 A1**
(43) Date of publication of application: **07.05.2025**
(21) Application number: 23852164.5
(22) Date of filing: 30.03.2023
(51) Int. Cl.: A61M 1/02, A61M 1/36

(54) **SYSTEM FOR RECOVERING ANTIGEN IN SOLVENT AND METHOD FOR RECOVERING ANTIGEN IN SOLVENT**

(30) Priority: 12.08.2022 JP 2022128953
(71) Applicant: GoGen Technologies, Ltd., Tokyo 108-0014 (JP)
(72) Inventor: NODA, Youhei, Tokyo 103-6128 (JP); NOGUCHI, Tomohiro, Tokyo 103-6128 (JP); NAGANO, Takashi, Tokyo 103-6128 (JP); UEDA, Mitsuyoshi, Takarazuka-shi, Hyogo 665-0033 (JP); AOKI, Wataru, Kyoto-shi, Kyoto 606-8393 (JP)
(74) Representative: TBK
(86) International application number: PCT/JP2023/013117
(87) International publication number: WO 2024/034178

(57) **Abstract**

This system (A) for recovering an antigen in a solvent comprises charged particles, a mixing unit (4), and a recovery unit (7), wherein: the charged particles each contain 3-70 mol% of a cationic lipid, 1-20 mol% of a PEG-containing lipid, and 20-50 mol% of cholesterol; the surfaces of the charged particles are modified with antibodies; the average zeta potential of the charged particles is -20 to +15 mV; the mixing unit (4) is configured such that the charged particles are added to a solvent (C) and the charged particles and the solvent (C) are stirred in the mixing unit (4); the recovery unit 7 is configured to recover the charged particles mixed with the solvent (C); and the recovery unit (7) is an anionic adsorber that includes an adsorption carrier having a surface on which a compound having an anionic functional group is immobilized.

## Description

### Technical Field

The present invention relates to a system for recovering an antigen in a solvent and a method for recovering an antigen in a solvent.

The present application claims priority based on Japanese Patent Application No. 2022-128953 filed in Japan on August 12, 2022, the contents of which are incorporated herein by reference.

### Background Art

As a method for effectively removing blood components (low-molecular-weight compounds, proteins, nucleic acids, viral particles, bacterial cells, and the like) that may be harmful to health, there is a demand for methods that are more effective and safer.

Examples of the substance to be removed include substances that cause diseases such as urea, creatinine, uric acid, β2 microglobulin, LDL cholesterol, abnormal antibodies in immune diseases, viruses, bacteria, fungi, and cancer cells. In addition, examples thereof include cytokines such as endotoxin and IL-6, heavy metal ions, and the like.

As a method for removing specific components from the blood, there are a method for administering a drug and removing specific components based on the action mechanism of the drug, and a direct removal technique which is a method for directly removing the specific components from the blood.

Examples of the former include the use of antibiotics against pathogenic bacteria and the use of antiviral agents against pathogenic viruses.

Examples of the latter direct removal technique include a magnetic separation type using antibody-modified magnetic beads.

In the direct removal technique, blood is taken from the inside of the living body to the outside of the living body, and the blood is subjected to a predetermined treatment to selectively remove a specific component. The blood that has been treated for removal is returned to the living body. The direct removal technique is relatively easy to develop because the action mechanism is simple. In the direct removal technique, it is expected that the effect can be obtained in a relatively short period of time.

As a magnetic separation type direct removal technique using antibody-modified magnetic beads, a technique described in Non-Patent Literature 1 is known.

### Citation List

### Non-Patent Literature

J. H. Kang et al., small, 2015, 11, No. 42, 5657-5666

### Summary of Invention

### Technical Problem

In a system for removing antigens from blood, high biocompatibility is one of the important indices.

In existing magnetic separation types including those described in Non-Patent Literature 1, magnetic beads made of an inorganic substance are used. These inorganic substances are substances that do not originally exist in the body, and are not metabolized in the living body.

The present disclosure provides a system and a method for efficiently removing specific components in a solvent using a medium having higher biocompatibility than magnetic beads.

### Solution to Problem

In order to solve the above problems, the present disclosure includes the following aspects.

A system for recovering an antigen in a solvent including: charged particles; a mixing unit; and a recovery unit, in which
the charged particles each contain 3 to 70 mol% of a cationic lipid, 1 to 20 mol% of a PEG-containing lipid, 20 to 50 mol% of cholesterol, and 0 to 65 mol% of a neutral lipid, surfaces of the charged particles are modified with antibodies, an average zeta potential of the charged particles is -20 to +15 mV,
the mixing unit is configured such that the charged particles are charged to a solvent and the charged particles and the solvent are stirred in the mixing unit, the recovery unit is configured to recover the charged particles mixed with the solvent, and the recovery unit is an anionic adsorber that includes an adsorption carrier having a surface on which a compound having an anionic functional group is immobilized.

A method for recovering an antigen in a solvent including: a step of charging charged particles into a solvent and stirring the charged particles; and a step of recovering the charged particles mixed with the solvent, in which the charged particles each contain 3 to 70 mol% of a cationic lipid, 1 to 20 mol% of a PEG-containing lipid, 20 to 50 mol% of cholesterol, and 0 to 65 mol% of a neutral lipid, surfaces of the charged particles are modified with antibodies, an average zeta potential of the charged particles is -20 to +15 mV, and the charged particles mixed with the solvent are recovered by electrostatic adsorption via an adsorption carrier having a surface on which a compound having an anionic functional group is immobilized.

### Advantageous Effects of Invention

According to the system for recovering an antigen in a solvent and the method for recovering an antigen in a solvent according to the aspect of the present disclosure, a predetermined component (antigen) in a solvent (for example, blood) can be efficiently removed using a highly biocompatible medium.

### Brief Description of Drawings

Fig. 1 is a schematic diagram of an example of a system for recovering an antigen in a solvent according to an embodiment of the present disclosure.
Fig. 2 is a schematic diagram of charged particles used in the system for recovering an antigen in a solvent according to the embodiment of the present disclosure.
Fig. 3 is a flowchart showing steps of a method for recovering an antigen in a solvent according to the embodiment of the present disclosure.

### Description of Embodiments

Hereinafter, aspects for carrying out the present disclosure will be described in detail with reference to the drawings as appropriate. In the drawings used in the following description, in order to facilitate understanding of features, features may be illustrated in an enlarged manner for convenience, and dimensional ratios and the like of each component may be different from actual ones. The materials, dimensions, and the like exemplified in the following description are examples, and the present disclosure is not limited thereto, and can be appropriately changed and implemented within the scope of achieving the effects of the present disclosure.

### (System for Recovering Antigen in Solvent)

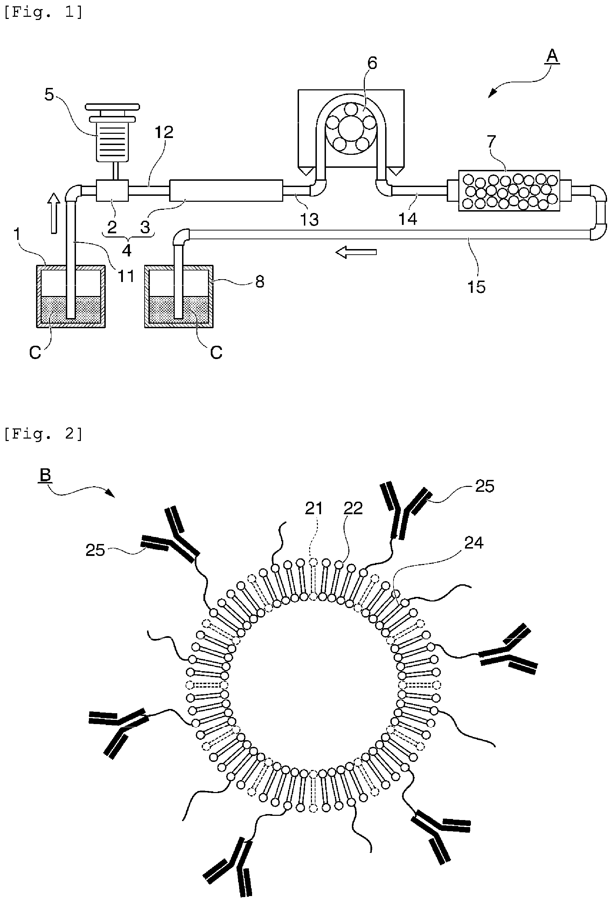

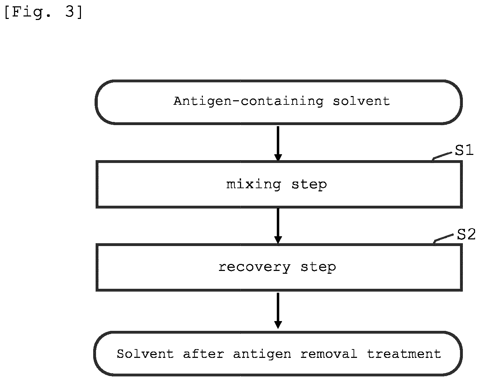

Fig. 1 is a schematic diagram of an example of a system for recovering an antigen in a solvent according to an embodiment of the present disclosure. Fig. 2 is a schematic diagram of charged particles used in the system for recovering an antigen in a solvent according to the embodiment of the present disclosure. Fig. 3 is a flowchart showing steps of a method for recovering an antigen in a solvent according to the embodiment of the present disclosure.

The system for recovering an antigen in a solvent A shown in Fig. 1 includes charged particles B, a mixing unit 4, and a recovery unit 7.

The charged particles B each contain 3 to 70 mol% of a cationic lipid, 1 to 20 mol% of a PEG-containing lipid, and 20 to 50 mol% of cholesterol.

The surfaces of the charged particles B are modified with antibodies 25, and the charged particles B have an average zeta potential of -20 to +15 mV.

The mixing unit 4 is configured such that the charged particles B are added to a solvent C and the charged particles B and the solvent C are stirred in the mixing unit 4.

The recovery unit 7 is configured to recover the charged particles B mixed with the solvent C.

The recovery unit 7 is an anionic adsorber that includes an adsorption carrier having a surface on which a compound having an anionic functional group is immobilized.

The system for recovering an antigen in a solvent A further includes a first tank 1, a pump 6, and a second tank 8 in addition to the essential components described above.

The mixing unit 4 may include a charged particles charging unit 2 and a mixer 3.

The system for recovering an antigen in a solvent A may further include a flow paths 11 to 15.

According to the system for recovering an antigen in a solvent A, it is possible to efficiently remove an antigen in a solvent using a medium having higher biocompatibility than the conventional technique.

### <Charged Particles>

Fig. 2 is a schematic diagram of charged particles used in the system for recovering an antigen in a solvent according to the embodiment of the present disclosure.

The charged particles B are liposomes containing a cationic lipid 21, a neutral lipid 22, a PEG-containing lipid 24, and an antibody 25.

The cationic lipid 21 is a positively charged lipid. Examples of the lipid that can be used as the cationic lipid 21 include DOTAP (1,2-dioleoyl-3-trimethylammonium-propane (chloride salt)), DOTMA (N-[1-(2,3-dioleoyloxy)propyl] -N,N,N-trimethylammonium chloride), DC-Chol (3-3-N-(N',N'-dimethylmethane) carbamoyl cholesterol), and DMRIE (dimyristyloxypropyl-3-dimethyl-hydroxyethyl ammonium) .

Among the cationic lipids, dendron lipids, DOSPER (1,3-dioleoyloxy-2-(6-carboxy-spermyl)-propylarnide), DOSPA (2,3-dioleyloxy-N-[2-(sperminecarboxamido)ethyl]-N,N-dimethyl-1 to propanaminium trifluoroacetate), and the like are not suitable as the cationic lipid 21 as a constituent component of the charged particles B because they decrease antibody activity due to a strong electrostatic interaction between the positive charge and the antibody.

The content of the cationic lipid 21 in the charged particles B is 3 to 70 mol%. When the content of the cationic lipid 21 is out of the above range, it is difficult to set the average zeta potential within a predetermined range to be described later.

In addition, when the content of the cationic lipid 21 is out of the above range, the electrostatic interaction with the adsorption simple substance included in the anionic adsorber as the recovery unit 7 becomes insufficient, and the recovery efficiency decreases.

The content of the cationic lipid 21 in the charged particles B may be 10 to 60 mol% or 25 to 55 mol%.

The type and content of the component (cationic lipids, neutral lipids, PEG-containing lipids, cholesterol, and the like) in the charged particle B can be identified and measured by a method such as high performance chromatography, thin layer chromatography, or mass spectrometry (LC-MS).

The neutral lipid 22 is a lipid that is neither positively nor negatively charged or a lipid containing equal amounts of positive charges and negative charges. Examples of the lipid that can be used as the neutral lipid 22 include DSPC (1, 2-distearoyl-sn-glycero-3 phosphoethanolamine), HSPC (hydrogenated soy phosphatidylcholine), DPPC (1,2-dipalmitoyl-sn-glycero-3-phosphocholine), and DOPE (dioleoyl phosphatidylethanolamine).

The content of the neutral lipid 22 in the charged particles B is 0 to 65 mol%. When the content of the neutral lipid is out of the above range, it is difficult to adjust the liposome, set the average zeta potential within a predetermined range to be described later, and the like.

The content of the neutral lipid 22 in the charged particles B may be 0 to 30 mol% or 0 to 25 mol%.

The PEG-containing lipid 24 is a lipid in which predetermined polyethylene glycol (PEG) is covalently bonded.

As PEG of the PEG-containing lipid 24, PEG having a molecular weight of 1,000 to 6,000 Da can be used. The molecular weight of PEG may be 1,800 to 5,500 Da or 2,000 to 3,000 Da. More specifically, the PEG may be PEG 2000 or PEG 5000.

As the PEG-containing lipid 24, DSPE-PEG (2000) Maleimide, DSPE-PEG (5000) Maleimide, DMG-PEG (1,2-dimyristoyl-rac-glycerol-3 methoxypolyethylene glycol), DSG-PEG (distearoyl-rac-glycerol-polyethylene glycol), or the like can be used.

The content of the PEG-containing lipid 24 in the charged particles B is 1 to 20 mol%. When the content of the PEG-containing lipid 24 is out of the above range, it is difficult to set the average zeta potential within a predetermined range to be described later as in the case of the cationic lipid.

In addition, when the content of the PEG-containing lipid 24 is out of the above range, the specificity of binding between the antigen in the solvent and the antibody presented on the surface of the charged particle B decreases when the charged particle B is mixed and dispersed with the solvent C, and the antigen recovery efficiency decreases.

The content of the PEG-containing lipid 24 in the charged particles B may be 6 to 14 mol% or 8 to 12 mol%.

The antibody 25 in the charged particles B is an antibody having, as an antigen, a substance to be removed from the solvent.

The antibody 25 may be any antibody as long as the antibody has a predetermined antigen specificity and can be used for preparation of the charged particles **B.** As long as the above conditions are satisfied, the antibody 25 may lack a part of the basic structure as a general antibody, or a part thereof may be replaced with a module having a function that the antibody does not originally have.

As the antibody 25, one obtained by artificially introducing an amino acid substitution, insertion, or deletion into a wild-type antibody can also be used.

As the antibody 25 in the charged particles B, a polyclonal antibody, a monoclonal antibody, an antibody produced by phage display, or the like can be used.

The content of the antibody 25 in the charged particles B is 5 to 1,000 µg/mg (antibody mass/charged particles). When the content of the antibody is less than 5 µg/mg, sufficient antigen recovery efficiency cannot be obtained. When the content of the antibody is more than 1,000 µg/mg, the characteristics (stability of charged particles and antigen recovery efficiency) as the charged particles B deteriorate.

The content of the antibody 25 in the charged particles B may be 20 to 200 µg/mg or 30 to 100 µg/mg.

The zeta potential is the potential difference between the slip plane in the electric double layer and a part sufficiently distant from the interface. The zeta potential is a parameter serving as an index of a dispersion state or the like of particles in the colloidal liquid.

The average zeta potential of the charged particles in the present disclosure is defined as a parameter required to specifically remove the antigen in the solvent.

The charged particles B have an average zeta potential of -20 to +15 mV. When the charged particles B have an average zeta potential of less than -20 mV, the recovery efficiency in the recovery unit 7, which is an anion adsorber, decreases even when the antigen and the antibody are bound to each other. When the charged particles B have an average zeta potential of more than +15 mV, non-specific binding between the antigen and the antibody due to electrostatic interaction is enhanced, and the antigen cannot be efficiently and specifically recovered.

The charged particles B may have an average zeta potential of -15 to +10 mV, or -10 to +5 mV.

The average zeta potential of the charged particles B (and the average zeta potential of the anion adsorber to be described later) can be measured by a commonly used method using the Smoluchowski equation.

For example, when the zeta potential of a predetermined liposome dispersion (HEPES 0.2 mM, NaCl 3 mM, pH 7.2) is measured, the measurement conditions are a measurement temperature of 25°C and a liposome concentration of 0.05 to 0.4 mg/ml.

In the zeta potential measurement setting, the nD20 of the liposome uses 1.590. Water is used as a buffer setting.

The charged particles B have an average particle size of 200 nm or less.

When the charged particles B have an average particle size of 200 nm or less, the diffusion coefficient of the charged particles B in the solvent C becomes a sufficiently small value. Therefore, the charged particles B rapidly bind to the antigen in the solvent by the antigen-antibody reaction. As a result, the antigen removal efficiency is improved.

The charged particles B may have an average particle size of 100 nm or less, or 60 nm or less.

The lower limit value of the average particle size of the charged particles is not particularly limited, but may be 30 nm.

The charged particles B can be prepared by a Bangham method, a method using a microfluidic channel, or the like. The charged particles B in the stage of use in the system for recovering an antigen in a solvent according to the present disclosure are a dispersion in which the charged particles are dispersed in a liquid. The charged particles B before use may be freeze-dried or may be in an antifreeze state to which sugars such as sucrose is added.

### <Solvent>

The solvent C is blood derived from a living body.

However, the solvent C is not necessarily limited to only blood. As long as the liquid is derived from a living body and contains a substance to be removed, a liquid other than blood can be used as the solvent C.

When blood is used, the collected blood can be used as it is, or pretreated blood can be used. Examples of the pretreatment include a treatment required to be provided to a system for recovering an antigen in a solvent (for example, removal of a substance that reduces the antigen recovery efficiency by the system for recovering an antigen in a solvent A).

In evaluating the performance of the system for recovering an antigen in a solvent, an artificially produced solvent may be used for the purpose of eliminating uncertain elements.

Examples of the artificially produced solvent include a PBS (physiological saline) solution containing a constant concentration of *E. coli* (antigen).

### <Mixing Unit>

The mixing unit 4 is configured such that the charged particles B are charged into the solvent C in the mixing unit 4, and the charged particles B and the solvent C are stirred.

The mixing unit 4 may be a sealable container having a predetermined volume (for example, 10 to 50 mL).

In this case, the charged particles B and the solvent C are mixed and stirred in the container.

For stirring, for example, a rotator having a rotating plate of which the rotation speed can be adjusted can be used. By immobilizing the container containing the charged particles B and the solvent C to the rotating plate and operating the rotator under the predetermined conditions (temperature, time, rotation speed, and the like), the charged particles B and the solvent can be mixed and stirred.

As shown in Fig. 1, the flow paths 11, 12, 13, 14, and 15 may be provided between the first tank 1, the mixing unit 4, the pump 6, the recovery unit 7, and the second tank 8, respectively, and the mixing unit 4 may be incorporated in a series of flows of the solvent C from the first tank to the second tank.

In this case, the mixing unit 4 includes the charged particles charging unit 2 and the mixer 3 connected to each other in series. The charged particles charging unit 2 is provided between the first tank and the mixer 3.

The charged particles charging unit 2 is a merging portion of three branches. The first one of the three is the first flow path 11. By the operation of the pump 6, the solvent C before treatment stored in the first tank 1 flows in. The second one of the three is the second flow path 12. By the operation of the pump 6, the solvent C in the charged particles charging unit 2 is sent from the charged particles B to the mixer 3.

The third one of the three is a charged particle charging flow path from a syringe 5. When the plunger of the syringe 5 is pushed, the charged particles B in the syringe are charged into the charged particles charging unit 2 at a constant speed.

The charged particles B (liposome dispersion) charged from the syringe 5 into the charged particles charging unit 2 has a charging speed of 1 to 10 µm/min.

In this case, the concentration of the charged particles B in the charged particles charging unit 2 is 0.01 to 2.0 mg/ml. When the charged particle concentration is less than 0.01 mg/ml, the charged particles B (liposome) introduced into the system are insufficient, and the antigen recovery efficiency decreases. When the charged particle concentration is more than 2.0 mg/ml, antibodies (free antibodies) not bound to the antigen are generated, and these saturate the adsorption carrier in the recovery unit 7 (anionic adsorber).

In that case, the antibody bound to the antigen is not sufficiently recovered by the recovery unit 7, and the recovery efficiency of the antigen is reduced.

The mixer 3 is configured to stir the solvent C and the charged particles B before the antigen recovery treatment merged at the charged particles charging unit 2. The mixer 3 may be a static mixer. By providing the mixer 3, the solvent C and the charged particles B before the antigen recovery treatment are efficiently and sufficiently mixed and stirred.

When the solvent C and the charged particles B before the antigen recovery treatment are sufficiently mixed and stirred before reaching the recovery unit, the mixer 3 can be omitted.

### <Recovery Unit>

The recovery unit 7 is configured to recover the charged particles B mixed with the solvent C. The recovery unit 7 is an anionic adsorber that includes an adsorption carrier having a surface on which a compound having an anionic functional group is immobilized.

The recovery unit 7 has a cylindrical shape with a predetermined length, and includes an outer wall and an internal space.

The length of the recovery unit 7 may be 10 to 300 mm, 30 to 200 mm, or 50 to 150 mm.

The inner diameter of the recovery unit 7 may be 10 to 100 mm, 15 to 70 mm, or 20 to 50 mm.

The volume of the internal space of the recovery unit 7 may be 0.5 to 750 ml, 1 to 500 ml, or 5 to 100 ml.

The recovery unit 7 includes an inlet for receiving the solvent C and the charged particles B mixed and stirred in the mixing unit 4, and an outlet through which the treated solvent C after passing through the inside of the recovery unit 7 is sent to the outside of the recovery unit.

The internal space of the recovery unit 7 is filled with an adsorption carrier having a surface on which a compound having an anionic functional group is immobilized.

The adsorption carrier includes a plurality of beads. The particle size of the beads is 10 to 500 µm. The particle size may be 20 to 300 µm or 40 to 200 µm.

The composition of the adsorption carrier is not particularly limited. For example, cellulose, agarose, silica gel, activated carbon, or the like can be used.

The anionic functional group of the compound fixed to the surface of the adsorption carrier may be a sulfate group, a carboxyl group, a phosphoric acid group, a sulfonic acid group, or the like.

The compound having an anionic functional group may be sodium dextran sulfate, sodium polyacrylate, sodium polyphosphate, sodium polystyrene sulfonate, sodium polyvinyl alcohol sulfate, or the like.

In addition, any compound that is relatively easy to immobilize on the surface of the adsorption carrier and can impart a negative charge to the surface of the adsorption carrier can be used as the compound.

The average zeta potential of the beads is -20 mV or less.

When the average zeta potential of the beads is -20 mV or less, the electrostatic interaction between the adsorption carrier and the charged particles B is enhanced, and the antigen recovery efficiency in the recovery unit 7 is improved.

The average zeta potential of the beads may be -30 mV or less.

### <Other Components of System for Recovering Antigen in Solvent A>

Hereinafter, configurations other than the charged particles B, the solvent C, the mixing unit 4, and the recovery unit 7 will be described.

The first tank 1 is a container in which the solution C before the antigen recovery (antigen removal) treatment is stored.

The second tank 8 is a container in which the solution C after the antigen recovery (antigen removal) treatment is stored.

The pump 6 is fluid pumping means, and transmits pressure for pumping the solution C from at least the mixing unit 4 to the recovery unit 7, to the solution C in the system.

As the pump 6, for example, a peristaltic pump suitable for aseptic liquid delivery can be used. A pump other than the peristaltic pump can also be used as long as aseptic liquid delivery is possible.

The pump 6 is configured to be able to control the flow rate of the solvent C (in the fifth flow path 15 to be described later) to 100 ml/min or less.

When the flow rate is more than 100 ml/min, the force due to the fluid increases, and the antigen adsorption efficiency in the recovery unit 7 (anionic adsorber) decreases.

The flow rate of the solvent C may be 0.15 to 90 ml/min or 1 to 50 ml/min.

The first tank and the charged particles charging unit 2 are connected to each other by the first flow path 11. The charged particles charging unit and the mixer 3 are connected to each other by the second flow path 12. The mixer 3 and the pump 6 are connected by the third flow path 13. The pump 6 and the recovery unit 7 are connected to each other by the fourth flow path 14. The recovery unit 7 and the second tank 8 are connected to each other by the fifth flow path 15.

### (Method for Recovering Antigen in Solvent)

Another embodiment of the present disclosure is a method for recovering an antigen in a solvent. Hereinafter, the method for recovering an antigen in a solvent will be described with reference to Fig. 3. Fig. 3 is a flowchart showing steps of a method for recovering an antigen in a solvent according to the embodiment of the present disclosure.

In the description of the method for recovering an antigen in a solvent, a part overlapping with the system for recovering an antigen in a solvent according to the above-described embodiment has the same content, and thus the description thereof will be omitted.

The method for recovering an antigen in a solvent includes a step S1 of charging the charged particles into a solvent and stirring the charged particles, and a step S2 of recovering the charged particles mixed with the solvent.

The charged particles each contain 3 to 70 mol% of a cationic lipid, 1 to 20 mol% of a PEG-containing lipid, and 20 to 50 mol% of cholesterol.

The surfaces of the charged particles are modified with antibodies.

The charged particles have an average zeta potential of -20 to +15 mV.

The charged particles mixed with the solvent are recovered by electrostatic adsorption via an adsorption carrier having a surface on which a compound having an anionic functional group is immobilized.

### <Mixing Step S1>

In the mixing step S1, the charged particles B are charged into the solvent C and stirred.

The charged particles B may be charged into solvent C by charging the charged particles B into a container containing the solvent C. As another means, a flow path for charging the charged particles into a flow path through which a solvent flows may be merged, and the charged particles B may be charged therefrom.

In the former case, a mixing container (for example, a plastic microtube) having a predetermined volume (for example, 0.1 ml to 2.0 ml) may be used as the container.

The concentration of the charged particles B in the mixing container may be 0.01 to 5.0 mg/ml. After the charged particles B are charged, the mixing container may be slowly turned upside down 5 to 10 times.

In the latter case, the concentration of the charged particles B in the charged particles charging unit 2 which is the merging part may be set to 0.01 to 2.0 mg/ml.

After the charged particles B are charged into the solvent C, the charged particles B and the solvent C are stirred.

When the charged particles B are charged into the mixing container containing the solvent C and the charged particles are charged, stirring can be performed using a rotator. The rotator includes a rotating plate that rotates along the circumferential direction.

The mixing container after charging the charged particles is immobilized to a rotating plate by a jig, and the content of the mixing container is stirred by rotating the rotating plate under the predetermined conditions (temperature, speed, time, and the like).

When a flow path for charging the charged particles into a flow path through which a solvent flows is merged and the charged particles B are charged therefrom, the mixer 3 (for example, a static mixer) may be provided downstream of the charged particles charging unit 2.

The mixer 3 may be omitted. In this case, the lengths of the flow path 13 and the flow path 14 from the charged particles charging unit 2 to the recovery unit 7 are set to a sufficient length. The length may be, for example, 0.01 to 1 m.

By setting the pump 6, the time during which the solvent C flows from the charged particles charging unit 2 to the recovery unit 7 may be set to 0.05 to 10 minutes.

### <Recovery Step S2>

In the recovery step S2, the charged particles mixed with the solvent are recovered.

In the recovery step S2, the charged particles B are adsorbed by electrostatic interaction between positive charging by the cationic lipid contained in the charged particles B and negative charging by the anionic functional group immobilized on the surface by the adsorption carrier.

The charged particles mixed with the solvent include charged particles bound to the antigen contained in the solvent C and charged particles not bound to the antigen.

In the recovery step S2, both charged particles are recovered.

Therefore, the recovery unit 7 has a sufficient charged particle recovery ability for the charged particles B to be charged not to be easily saturated with the charged particles.

### Examples

Hereinafter, the present invention will be described based on more detailed examples, but the present invention is not limited to these examples.

### (Example 1)

### (Evaluation Using PBS Containing E. coli as Solution)

First, *E. coli* was cultured and prepared.

In a clean bench, 10 ml of LB liquid medium was added to a 25 ml test tube, and a small amount (several µl) of seed culture solution of *E. coli* was added. Next, E. *coli* was cultured in a shaker at 37°C, 12 hours, and 140 rpm. 1 ml of the *E. coli* solution after culture was taken and dispensed into a 1.5 ml tube. The tube was centrifuged at 4,400 rpm for 5 minutes to sediment *E. coli* cells at the bottom of the tube, and the supernatant was removed. 1 ml of PBS buffer was added to the tube, and the *E. coli* cells sedimented to the bottom by pipetting was resuspended.

The steps from centrifugation to resuspension described above were repeated once more.

The concentration in the *E. coli* cells in the solution after the second resuspension was adjusted to 8 × 10⁸ CFU/ml. Thereafter, the *E. coli* suspension was diluted 1,000 times with PBS. The *E. coli* cells concentration at this time was 8 × 10⁵ CFU/ml. More specifically, the operation of adding 0.9 ml of PBS to 0.1 ml of the *E. coli* suspension to dilute 10 times was repeated 3 times.

Next, preparation of a liposome dispersion (charged particle-containing aqueous solution) was performed by a Bangham method. Specifically, the measurement was performed by the following method.

The constituent components of each liposome were stirred and dissolved in a mixed solution of chloroform and methanol at the specified ratio. The solvent was distilled off using an evaporator to produce a lipid film. The dispersion solution obtained from the lipid film was passed through a hydrophilic polycarbonate membrane and subjected to an extruder treatment to regulate the particle size. A reduced anti-E. *coli* antibody was added to the liposome dispersion. Then, the mixture was stirred overnight under refrigeration. Free antibodies were removed by ultrafiltration at room temperature in a solvent. The solution was recovered.

PBS was added to a previously prepared liposome dispersion (approximately 15 mg/ml) to adjust the concentration of the liposome to 1 mg/ml.

Next, preliminary mixing was performed.

0.2 ml of the liposome dispersion (1 mg/ml) prepared in the above step was added to a 1.5 ml tube, and 0.05 ml of the *E. coli* cell suspension (approximately 8 × 10⁵ CFU/ml) was added thereto. Thereafter, the tube was immobilized to the rotating plate of the rotator and stirred for 30 minutes or more (20 rpm). After stirring, 0.2 ml of the tube contents was taken using a pipette and added to PBS (5 ml) in a separately prepared container (volume: 5 ml). Thereafter, the container was slowly turned upside down approximately 10 times.

Next, a flow-through recovery test was performed.

A flow-through recovery test apparatus partially overlapping with the system for recovering an antigen in a solvent shown in Fig. 1 was assembled. The difference from Fig. 1 is that the mixing unit 4 is omitted, and the first tank stores a mixed solution (5 ml) of the liposome suspension and the *E. coli* cell suspension described above.

The inlet-side solution was suctioned with a peristaltic pump (suction pump), circulated through an anionic adsorber connected to a tube at 7.5 to 15 ml/h, and discharged to the outlet side. From each elapsed time (for example, 10 minutes/20 minutes/30 minutes) after the start of circulation, a sample was recovered approximately every 1 minute.

Next, the viable *E. coli* cell concentration of the recovered sample was measured.

0.1 ml of the outlet-side sample at each elapsed time from the start of circulation of the flow-through adsorption recovery test was collected, and added to 0.9 ml of PBS to be diluted 10 times.

0.1 ml of the sample diluted 10 times was uniformly applied to the surface of the agar medium, and the agar medium was statically cultured at 35°C for 22 to 24 hours.

On the next day, the number of colonies formed was measured, and the viable cell concentration was calculated from the following formula. Three agar media were used for each sample, and the viable cell concentration was calculated from the average value thereof. Viable cell concentration (CFU/ml) = (number of colonies) × (1.0 ml/0.1 ml) × (dilution ratio (10 times))

The viable cell concentration of the inlet-side sample was calculated in the same manner.

The adsorption removal rate was calculated according to the following formula based on the number of viable E. *coli* cells in the inlet-side sample and the outlet-side sample. Adsorption removal rate (%) = 100 × {1 - (viable cell concentration of outlet-side sample/viable cell concentration of inlet-side sample)} Specific adsorption removal rate (%) = 100 × {1 - (Outlet-side viable cell concentration of E.coli antibody-modified liposome (Example)/Outlet-side viable cell concentration of control antibody-modified liposome (Comparative Example))}

Here, the same composition was used for the *E. coli* antibody-modified liposome and the control antibody-modified liposome.

A control antibody (isotype control) was used for a negative control that does not recognize *E. coli* as an antigen. Specifically, the same Rabbit IgG Isotype Control antibody as the isotype control of the *E. coli* antibody was used.

Table 1 shows the solvents, antigens, and cell concentrations used in Examples A1 to A13 and Comparative Examples A1 to A12.

Table 2 shows modified antibodies (type and concentration), compositions (type and content of each composition), and properties (average zeta potential and average particle size) of liposomes (charged particles) used in these Examples and Comparative Examples.

Table 3 shows premixing conditions, pump flow rates, solvents, compositions of the anionic adsorber (types of surface functional groups and anionic compounds), and properties (average zeta potential and average particle size) in these Examples and Comparative Examples.

Table 4 shows the adsorption removal rate and the specific adsorption removal rate as evaluations in these Examples and Comparative Examples.

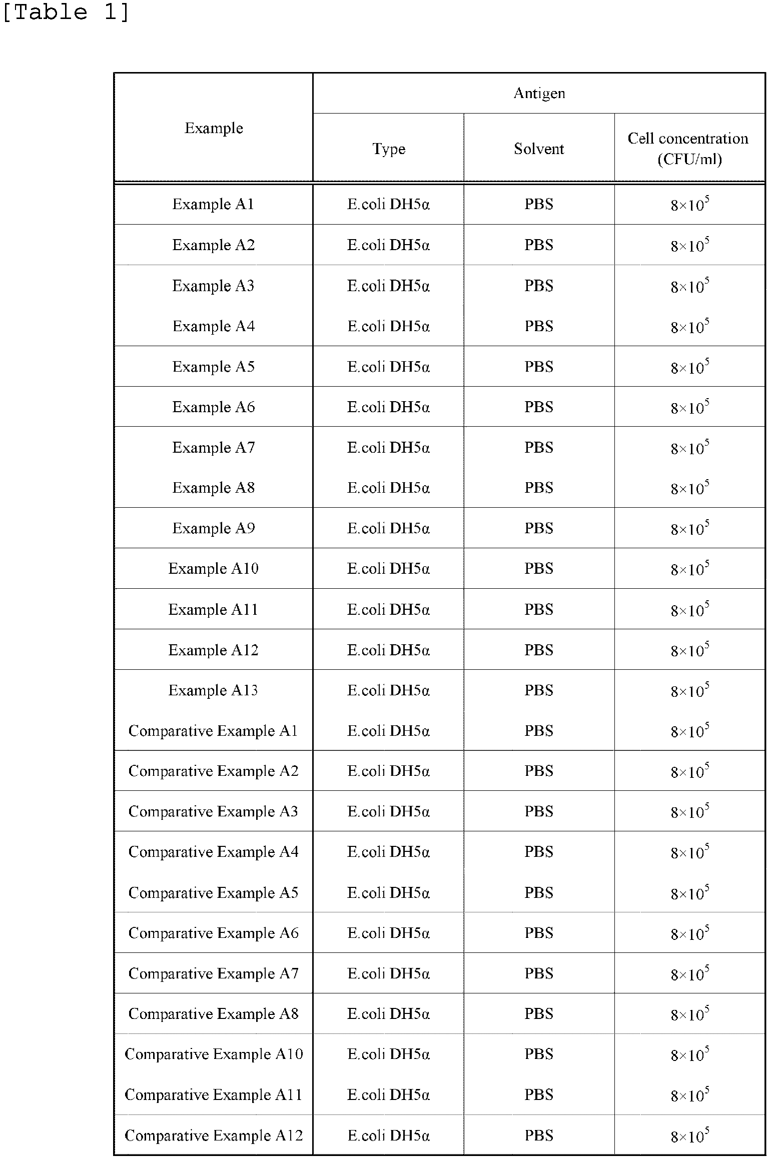

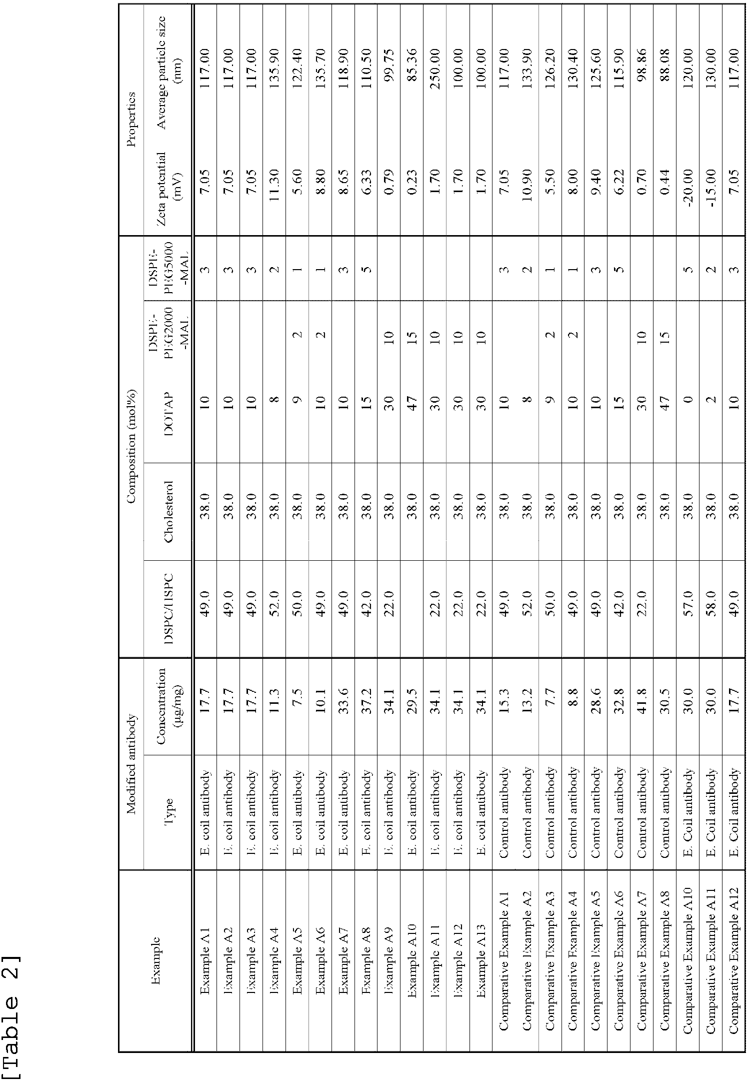

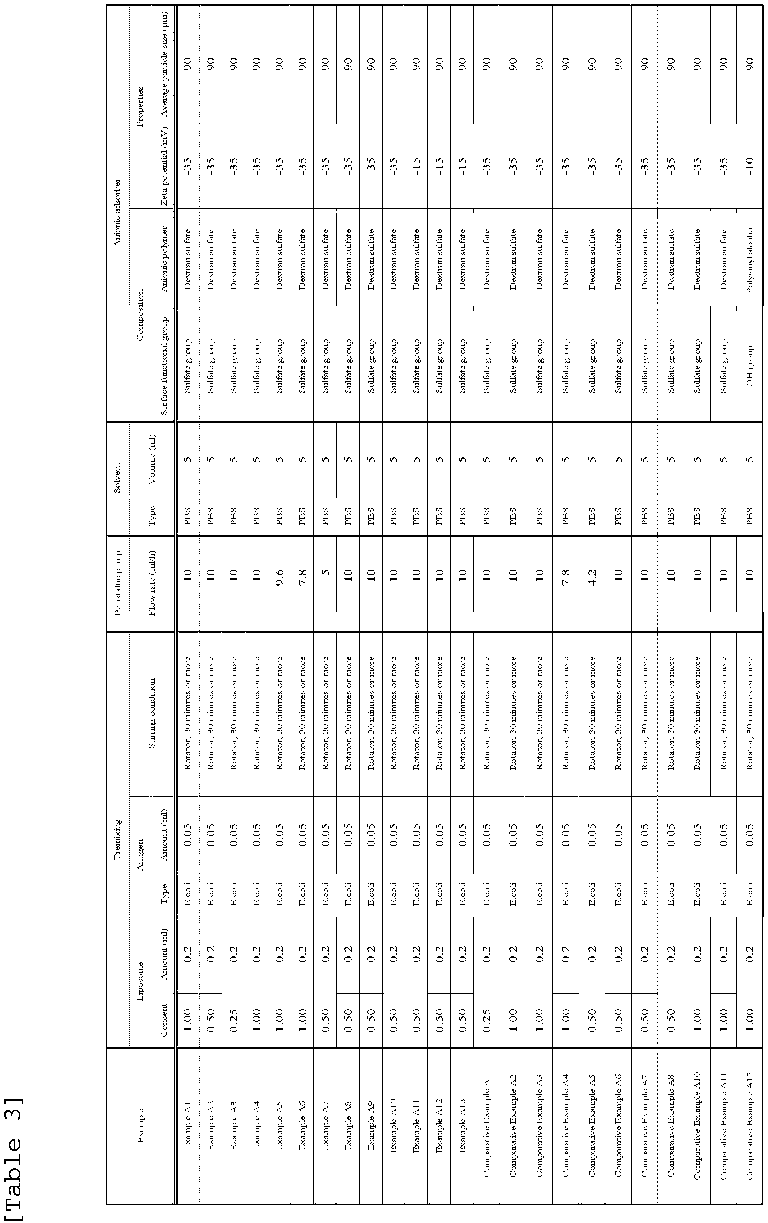

**[Table 4]**

| Example | Adsorption removal rate (1-(Cout/Cin)) | | | Specific adsorption removal rate (1-(C1/C2)) |
|---|---|---|---|---|
| | Elapsed time (min) | | | |
| | 10 | 20 | 30 | |
| Example A1 | 100.00% | 100.00% | 99.50% | 99.80% |
| Example A2 | 94.30% | 93.50% | 90.40% | 87.80% |
| Example A3 | 69.30% | 43.90% | 13.60% | - |
| Example A4 | 92.10% | 94.70% | 93.40% | 87.00% |
| Example A5 | 93.80% | 57.80% | 83.30% | 65.60% |
| Example A6 | 83.10% | 41.30% | 36.50% | 37.00% |
| Example A7 | 95.20% | 9420% | 9420% | 92.30% |
| Example A8 | 99.80% | 99.30% | 99.10% | 99.00% |
| Example A9 | 99.00% | 100.00% | 99.50% | 99.20% |
| Example A10 | 76.60% | 76.00% | 76.00% | 75.20% |
| Example A11 | 80.00% | 90.00% | 80.00% | 90.00% |
| Example A12 | 70.00% | 60.00% | 70.00% | 72.00% |
| Example A13 | 55.50% | 50.00% | 45.00% | 56.00% |
| Comparative Example A1 | 54.90% | 46.50% | 36.20% | - |
| Comparative Example A2 | 90.80% | 73.40% | 1.70% | - |
| Comparative Example A3 | 58.80% | 42.00% | 31.30% | - |
| Comparative Example A4 | 19.90% | 34.80% | 1.20% | - |
| Comparative Example A5 | 36.00% | 39.10% | 35.30% | - |
| Comparative Example A6 | 47.10% | 26.50% | 22.60% | - |
| Comparative Example A7 | 57.90% | 37.00% | 37.40% | - |
| Comparative Example A8 | 25.40% | 43.60% | 27.80% | - |
| Comparative Example A10 | 11.00% | 6.00% | -3.00% | - |
| Comparative Example A11 | 5.00% | 7.00% | -5.00% | - |
| Comparative Example A12 | -10.00% | 7.00% | 4.00% | - |

### (Examples A1 to A13 and Comparative Examples A1 to A12)

In Examples A1 to A13 satisfying the conditions shown in Tables 1 to 3, by using appropriate liposomes (charged particles), mixing units, and recovery units, a remarkably high adsorption removal rate could be obtained as compared with Comparative Examples A1 to A12.

Comparative Example A1 to Comparative Example A12 generally correspond to Examples A3 to A11. In Comparative Examples A1 to A12, control antibodies are used as antibodies, and the specificity of the recovery rates obtained in Examples can be seen by comparing these corresponding Examples with Comparative Examples.

From the results of Examples A1 to A3, it is found that the adsorption removal rate decreases as the input amount of the liposome decreases.

In Comparative Examples A10 and A11, the content of the cationic lipid in the liposome was as low as 0 mol% and 2 mol%, respectively, and the adsorption removal rate was as extremely low as -5% to 11%, despite the use of an E. *coli* antibody.

In Comparative Example A12, a hydroxyl group is used as the surface functional group of the anionic adsorber, and polyvinyl alcohol is used as the nonionic polymer (nonionic compound). The average zeta potential of the anionic adsorber in Comparative Example A12 was -10 mV, which was extremely low, and the adsorption removal rate was as extremely low as -10% to 7%, despite the use of an *E. coli* antibody.

As a result of evaluating the effect of the system for recovering an antigen in a solvent according to the present disclosure using a PBS solution containing *E. coli* as a solvent, it was shown that the antigen in the solvent can be efficiently recovered using highly biocompatible liposomes.

### (Example 2)

### (Evaluation Using Bovine Blood Containing E. coli as Solution)

The effect of the system for recovering an antigen in a solvent according to the present disclosure was evaluated almost in the same manner as in Example **1.** Example 2 is different in that the PBS solution used as a solvent containing an antigen in Example 1 was replaced with bovine blood (pretreated) or human blood (pretreated). The description of other identical parts will be omitted.

Bovine blood and human blood include substances having an action of lysing bacteria. When these blood samples are directly subjected to the evaluation of the present Example, not only the decrease in the number of viable *E. coli* cells in the solvent by the system for recovering an antigen in a solvent but also the decrease due to this lytic action are included, and thus these blood samples are not suitable as the evaluation target.

For the above reasons, in the present example in which bovine blood or human blood is subjected to a test as a solvent containing an antigen, these liquids were pretreated to avoid the above problems.

Specifically, bovine blood or human blood was passed through an anionic adsorber at 15 ml/h using a flow-through apparatus to partially remove components related to the innate immune function contained in the bovine blood.

Next, *E. coli* was cultured.

In a clean bench, 10 ml of LB liquid medium was added to a 25 ml test tube, and a small amount (several µl) of seed culture solution of *E. coli* was added. Next, E. *coli* was cultured in a shaker at 37°C, for 12 hours, at 140 rpm.

1 ml of the *E. coli* solution after culture was taken and dispensed into a 1.5 ml tube.

The tube was centrifuged at 4,400 rpm for 5 minutes to sediment *E. coli* cells at the bottom of the tube, and the supernatant was removed. 1 ml of PBS buffer was added to the tube, and the *E. coli* cells separated to the bottom by pipetting was resuspended.

The concentration in the *E. coli* cells in the solution after the second resuspension was adjusted to 8 × 10⁸ CFU/ml. Thereafter, the *E. coli* suspension was diluted 1,000 times with PBS. The *E. coli* cells concentration at this time was 8 × 10⁵ CFU/ml. More specifically, the operation of adding 0.9 ml of PBS to 0.1 ml of the *E. coli* suspension to dilute 10 times was repeated 3 times.

The liposome dispersion (charged particle-containing aqueous solution) was prepared in the same manner as in Example 1.

Pretreated bovine blood was added to a prepared liposome dispersion (approximately 15 mg/ml) to adjust the concentration of the liposome to 0.5 to 2 mg/ml.

The premixing, the flow-through recovery test, and the measurement of the viable *E. coli* cell concentration in the recovered sample were performed in the same manner as in Example 1 except that the pretreated bovine blood or the pretreated human blood was used instead of PBS. Table 5 shows the solvents, antigens, and cell concentrations used in Examples B1 to B13 and Comparative Examples B1 to B8.

Table 6 shows modified antibodies (type and concentration), compositions (type and content of each composition), and properties (average zeta potential and average particle size) of liposomes (charged particles) used in these Examples and Comparative Examples.

Table 7 shows premixing conditions, pump flow rates, solvents, compositions of the anionic adsorber (types of surface functional groups and anionic compounds), and properties (average zeta potential and average particle size) in these Examples and Comparative Examples.

Table 8 shows the adsorption removal rate and the specific adsorption removal rate as evaluations in these Examples and Comparative Examples.

**[Table 5]**

| Example | Antigen | | |
|---|---|---|---|
| | Type | Solvent | Cell concentration (CFU/ml) |
| Example B1 | E.coli DH5α | Filtered bovine blood | 8×10⁵ |
| Example B2 | E.coli DH5α | Filtered bovine blood | 8×10⁵ |
| Example B3 | E.coli DH5α | Filtered bovine blood | 8×10⁵ |
| Example B4 | E.coli DH5α | Filtered bovine blood | 8×10⁵ |
| Example B5 | E.coli DH5α | Filtered bovine blood | 8×10⁵ |
| Example B6 | E.coli DH5α | Filtered human blood | 8×10⁵ |
| Example B7 | E.coli DH5α | Filtered bovine blood | 8×10⁵ |
| Example B8 | E.coli DH5α | Filtered bovine blood | 8×10⁵ |
| Example B9 | E.coli DH5α | Filtered bovine blood | 8×10⁵ |
| Example B10 | E.coli DH5α | Filtered bovine blood | 8×10⁵ |
| Example B11 | E.coli DH5α | Filtered human blood | 8×10⁵ |
| Comparative Example B1 | E.coli DH5α | Filtered bovine blood | 8×10⁵ |
| Comparative Example B2 | E.coli DH5α | Filtered bovine blood | 8×10⁵ |
| Comparative Example B3 | E.coli DH5α | Filtered human blood | 8×10⁵ |
| Comparative Example B4 | E.coli DH5α | Filtered bovine blood | 8×10⁵ |
| Comparative Example B5 | E.coli DH5α | Filtered human blood | 8×10⁵ |
| Comparative Example B6 | E.coli DH5α | Filtered bovine blood | 8×10⁵ |
| Comparative Example B7 | E.coli DH5α | Filtered bovine blood | 8×10⁵ |
| Comparative Example B8 | E.coli DH5α | Filtered human blood | 8×10⁵ |

**[Table 6]**

| Example | Modified antibody | | Composition (mol%) | | | | | Properties | |
|---|---|---|---|---|---|---|---|---|---|
| | Type | Concentration (µg/mg) | DSPC/HSPC | Cholesterol | DOTAP | DPSE-PEG2000 -MAL | DPSE-PEG5000 -MAL | Zeta potential (mV) | Average particle size (nm) |
| Example B1 | E.coli antibody | 37.2 | 42 | 38 | 15 | | 5 | 6.300 | 110.5 |
| Example B2 | E.coli antibody | 34.1 | 22 | 38 | 30 | 10 | | 1.700 | 103.7 |
| Example B3 | E.coli antibody | 34.1 | 22 | 38 | 30 | 10 | | 1.700 | 103.7 |
| Example B4 | E.coli antibody | 34.1 | 22 | 38 | 30 | 10 | | 1.700 | 103.7 |
| Example B5 | E.coli antibody | 34.1 | 22 | 38 | 30 | 10 | | 1.700 | 103.7 |
| Example B6 | E.coli antibody | 29.5 | | 38 | 47 | 15 | | 1.840 | 101.8 |
| Example B7 | E.coli antibody | 39.1 | 22 | 38 | 30 | 10 | | 0.785 | 98.8 |
| Example B8 | E.coli antibody | 17.7 | 49 | 38 | 10 | | 3 | 7.050 | 117.0 |
| Example B9 | E.coli antibody | 11.3 | 52 | 38 | 8 | | 2 | 11.300 | 135.9 |
| Example B10 | E.coli antibody | 39.1 | 22 | 38 | 30 | 10 | | 0.785 | 232.0 |
| Example B11 | E.coli antibody | 29.5 | | 38 | 47 | 15 | | 1.840 | 245.0 |
| Comparative Example B1 | Control antibody | 32.8 | 42 | 38 | 15 | | 5 | 6.220 | 115.9 |
| Comparative Example B2 | Control antibody | 31.6 | 22 | 38 | 30 | 10 | | 2.000 | 109.0 |
| Comparative Example B3 | Control antibody | 25.2 | | 38 | 47 | 15 | | 2.030 | 108.6 |
| Comparative Example B4 | Control antibody | 41.8 | 22 | 38 | 30 | 10 | | 0.740 | 98.9 |
| Comparative Example B5 | Control antibody | 20.0 | 49 | 38 | 10 | | 3 | 7.000 | 120.0 |
| Comparative Example B6 | Control antibody | 10.0 | 52 | 38 | 8 | | 2 | 12.000 | 140.1 |
| Comparative Example B7 | Control antibody | 39.1 | 22 | 38 | 30 | 10 | | 0.800 | 232.0 |
| Comparative Example B8 | Control antibody | 29.5 | | 38 | 47 | 15 | | 2.100 | 245.0 |

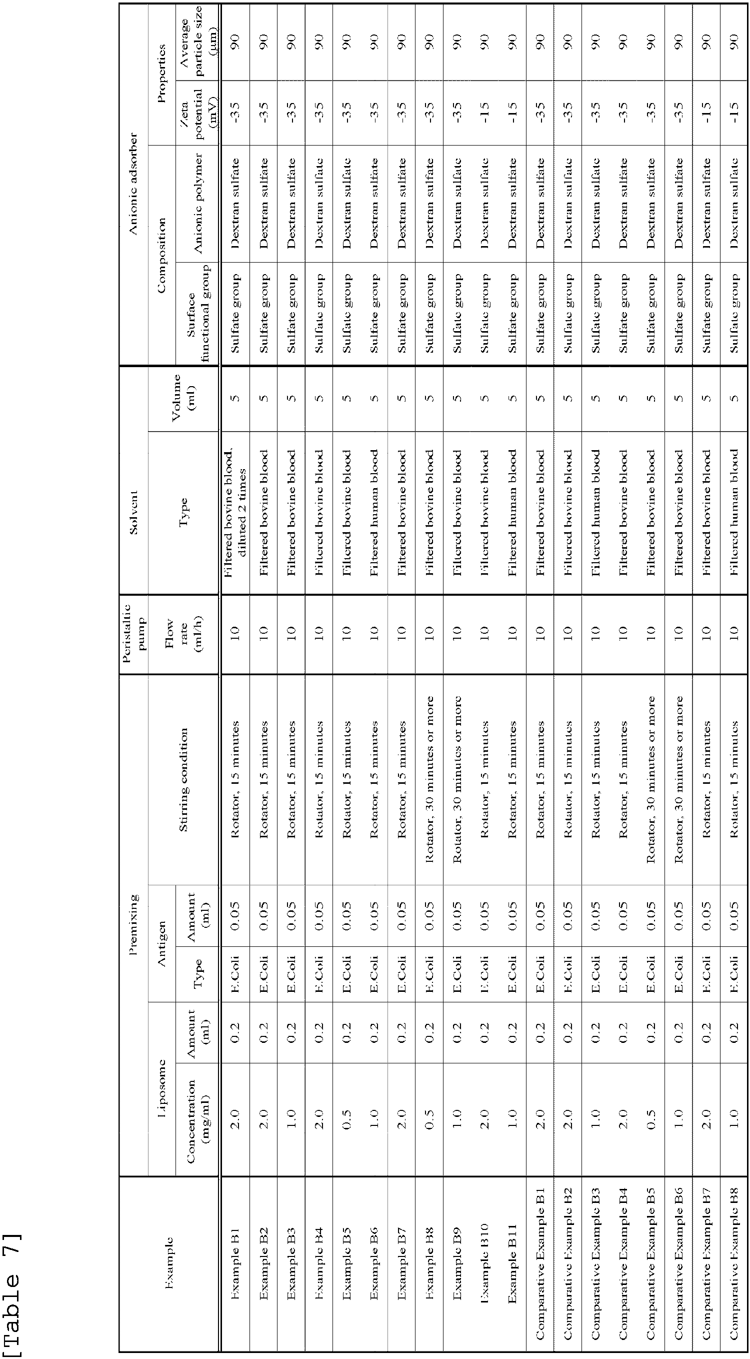

**[Table 8]**

| Example | Adsorption removal rate (1-(Cout/Cin)) | | | Specific adsorption removal rate (1-(C1/C2)) |
|---|---|---|---|---|
| | Elapsed time (min) | | | |
| | 10 | 20 | 30 | |
| Example B1 | 46.0% | 43.5% | 31.0% | 8.6% |
| Example B2 | 69.8% | 69.1% | 69.1% | 52.9% |
| Example B3 | 91.6% | 91.6% | 94.4% | 88.2% |
| Example B4 | 71.9% | 78.6% | 70.0% | 71.7% |
| Example B5 | 78.4% | 85.1% | 84.4% | 70.7% |
| Example B6 | 56.2% | 28.8% | 25.1% | 21.1% |
| Example B7 | 54.9% | 52.6% | 29.7% | 43.8% |
| Example B8 | 20.0% | 30.0% | 40.0% | 10.0% |
| Example B9 | 10.0% | 15.0% | 15.0% | 9.0% |
| Example B10 | 5.0% | 7.0% | 10.0% | 7.0% |
| Example B10 | 10.0% | 5.0% | 6.0% | 6.0% |
| Comparative Example B1 | 27.9% | 33.3% | 28.1% | - |
| Comparative Example B2 | 36.7% | 29.6% | 32.6% | - |
| Comparative Example B3 | 11.1% | 14.8% | -4.8% | - |
| Comparative Example B4 | 13.3% | 9.8% | 14.8% | - |
| Comparative Example B5 | 5.0% | 6.0% | 10.0% | - |
| Comparative Example B6 | 2.0% | 3.0% | 4.0% | - |
| Comparative Example B7 | 3.0% | 1.0% | 2.0% | - |
| Comparative Example B8 | 2.0% | 1.3% | 4.4% | - |

### (Examples B1 to B11 and Comparative Examples B1 to B8)

In Examples B1 to B11 satisfying the conditions shown in Tables 5 to 7, by using appropriate liposomes (charged particles), mixing units, and recovery units, a high adsorption removal rate could be obtained as compared with Comparative Examples B1 to B8.

In Comparative Examples B1 to B8, control antibodies are used as antibodies, and the specificity of the recovery rates obtained in Examples can be seen by comparing these corresponding Examples with Comparative Examples.

In Examples B8 and B9, the content of the PEG-containing lipid in the liposome was mainly lower than that in other Examples, and therefore it is presumed that the adsorption removal rate was reduced.

In Examples B10 to B11, the particle size of the liposome was mainly larger than that in other Examples, and the average zeta potential of the anion adsorber was lower than that in other Examples, and thus it is presumed that the adsorption removal rate was reduced.

As a result of evaluating the effect of the system for recovering an antigen in a solvent according to the present disclosure using a bovine or human blood containing *E. coli* as a solvent, it was shown that the antigen in the solvent can be efficiently recovered using highly biocompatible liposomes.

### (Example 3)

### (Evaluation of Integrated System for Recovering Antigen in Solvent)

In Example 3, the system for recovering an antigen in a solvent according to the present disclosure in which the mixing unit 4 is integrated was evaluated.

Example 3 is different from Example 1 in that the mixing unit 4 configured as a separate container in Example 1 is incorporated into and integrated with a solvent circulation system of a system for recovering an antigen in a solvent. The description of other identical parts will be omitted.

Culturing and preparation of *E. coli* were performed in the same manner as in Example **1.**

A liposome dispersion (charged particle-containing aqueous solution) was prepared in the same manner as in Example **1.**

The apparatus shown in Fig. 1 was assembled, and the prepared cell-containing solvent (PBS, blood) was circulated at approximately 10 ml/h with a peristaltic pump and discharged to the outlet side. Samples were collected 20 minutes, 30 minutes, and 40 minutes after the start of circulation. Liposomes were introduced with the syringe 5 40 minutes after the start of circulation. Samples were collected after 60 minutes from the start of circulation.

The number of viable ***E.** coli* cells contained in the collected sample was measured in the same manner as in Example **1.**

For the evaluation of the system for recovering an antigen in a solvent, the adsorption removal rate after charging, as defined by the following formula, was also calculated. Adsorption removal rate after charging (%) = 100 × (Outlet-side viable cell concentration immediately before charging liposome)/ (Outlet-side viable cell concentration after charging liposomes)

Table 9 shows modified antibodies (type and concentration), compositions (type and content of each composition), and properties (average zeta potential and average particle size) of liposomes (charged particles) used in Examples C1 to C9 and Comparative Examples C1 and C2.

Table 10 shows the liposome concentration, the charging speed, and the concentration in the solvent after charging in these Examples and Comparative Examples. Table 10 further shows pump flow rates, solvents, compositions of the anionic adsorber (types of surface functional groups and anionic compounds), and properties (average zeta potential and average particle size).

Table 11 shows the adsorption removal rate after charging as evaluations in these Examples and Comparative Examples. When the adsorption removal rate after charging was 20% or more, it was determined that there was an adsorption increasing effect after charging liposomes, and the results are indicated by the symbol "∘",

**[Table 9]**

| Example | Modified antibody | | Composition (mol%) | | | | | Properties | |
|---|---|---|---|---|---|---|---|---|---|
| | Type | Concentration (µg/mg) | DSPC/ HSPC | Cholesterol | DOTAP | DSPE-PEG2000 -MAL | DSPE-PEG5000 -MAL | Zeta potential (mV) | Average particle size (nm) |
| Example C1 | E.coli antibody | 34.1 | 22 | 38 | 30 | 10 | | 1.700 | 103.70 |
| Example C2 | E.coli antibody | 34.1 | 22 | 38 | 30 | 10 | | 1.700 | 103.70 |
| Example C3 | E.coli antibody | 29.5 | | 38 | 47 | 10 | | 1.840 | 101.80 |
| Example C4 | E.coli antibody | 29.5 | | 38 | 47 | 10 | | 1.840 | 101.80 |
| Example C5 | E.coli antibody | 39.1 | 22 | 38 | 30 | 10 | | 0.785 | 98.75 |
| Example C6 | E.coli antibody | 39.1 | 22 | 38 | 30 | 10 | | 0.785 | 98.75 |
| Example C7 | E.coli antibody | 39.1 | 22 | 38 | 30 | 10 | | 0.785 | 98.75 |
| Example C8 | E.coli antibody | 39.1 | 22 | 38 | 30 | 10 | | 0.785 | 98.75 |
| Example C9 | E.coli antibody | 39.1 | 22 | 38 | 30 | 10 | | 0.785 | 98.75 |
| Comparative Example C1 | Control antibody | | 22 | 38 | 30 | 10 | | 0.740 | 98.86 |
| Comparative Example C2 | - | | | | | | | | |

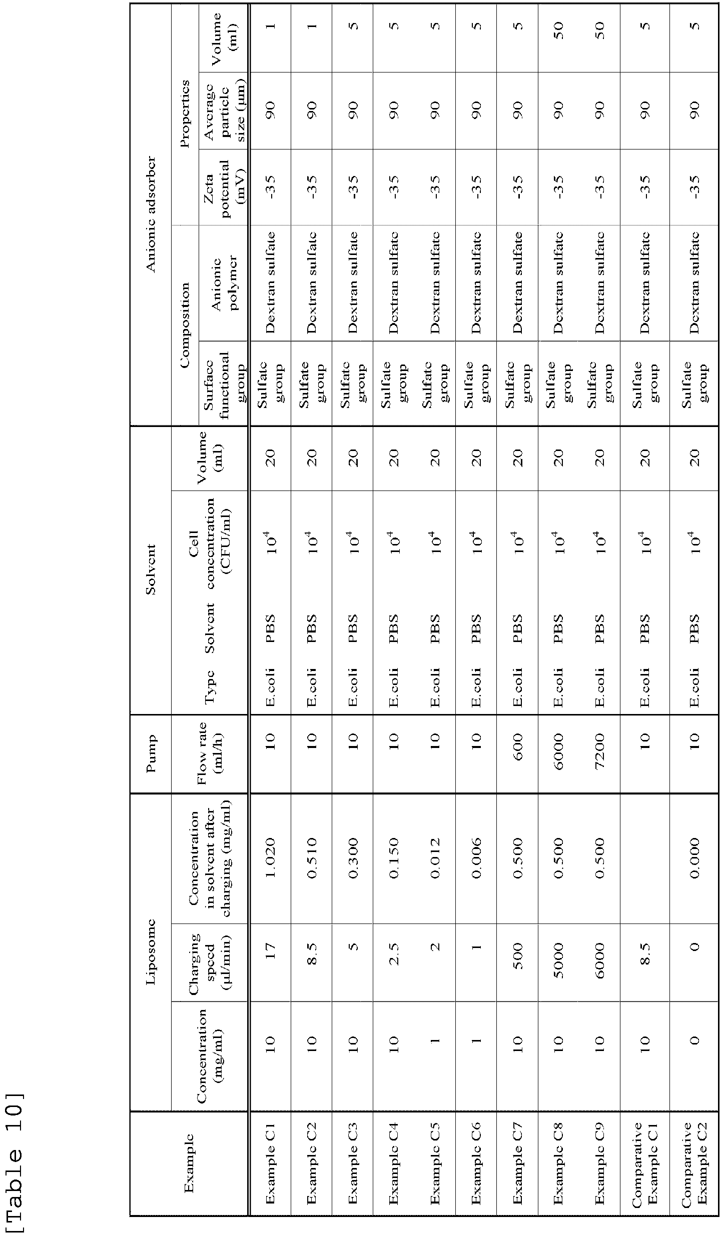

**[Table 11]**

| Example | Adsorption removal rate after charging | |
|---|---|---|
| | Elapsed time (min) | |
| | Adsorption increase effect after charging liposomes | Adsorption removal rate after charging (1-(Cimmediately beforecharging/Cafter charging)) |
| Example C1 | ○ | 50.0% |
| Example C2 | ○ | 50.0% |
| Example C3 | ○ | 95.0% < |
| Example C4 | ○ | 95.0% < |
| Example C5 | ○ | 80.0% |
| Example C6 | ○ | 20.0% |
| Example C7 | ○ | 95.0% < |
| Example C8 | ○ | 50.0% |
| Example C9 | ○ | 20.0% |
| Comparative Example C1 | | 0.0% |
| Comparative Example C2 | | 0.0% |

### (Examples C1 to C9 and Comparative Examples C1 to C2)

In all of Examples C1 to C9, the adsorption removal rate after charging was improved by at least 20% or more as compared with Comparative Examples C1 and C2.

In Example C6, since the liposome charging concentration, the charging speed, and the concentration in the solvent after charging were lower than those in other Examples, it is presumed that the adsorption removal rate after charging was lower than that in other Examples.

In Example C9, since the liposome charging speed and the concentration in the solvent after charging were higher than those in other Examples, and the pump flow rate was extremely faster than that in other Examples, it is presumed that the adsorption removal rate after charging was lower than that in other Examples.

It was shown that the system for recovering an antigen in a solvent according to the present disclosure can efficiently recover an antigen in a solvent using a highly biocompatible medium even in an aspect in which the mixing unit 4 is integrated.

### Industrial Applicability

It is possible to efficiently remove a predetermined component (antigen) in a solvent (for example, blood) using a highly biocompatible medium.

### Reference Signs List

- A: System for recovering antigen in solvent
- 1: First tank
- 2: Charged particles charging unit
- 3: Mixer
- 4: Mixing unit
- 5: Syringe
- 6: Pump
- 7: Recovery unit
- 8: Second tank
- B: Charged particle (liposome)
- 21: Cationic lipid
- 22: Neutral lipid
- 24: PEG-containing lipid
- 25: Antibody
- C: Solvent (blood)

## Claims

1. A system for recovering an antigen in a solvent, comprising:
charged particles;
a mixing unit; and
a recovery unit, wherein
the charged particles each contain 3 to 70 mol% of a cationic lipid, 1 to 20 mol% of a PEG-containing lipid, 20 to 50 mol% of cholesterol, and 0 to 65 mol% of a neutral lipid,
surfaces of the charged particles are modified with antibodies,
an average zeta potential of the charged particles is -20 to +15 mV,
the mixing unit is configured such that the charged particles are charged to a solvent and the charged particles and the solvent are stirred in the mixing unit,
the recovery unit is configured to recover the charged particles mixed with the solvent, and
the recovery unit is an anionic adsorber that includes an adsorption carrier having a surface on which a compound having an anionic functional group is immobilized.

2. The system for recovering an antigen in a solvent according to claim 1, wherein
the anionic functional group is a sulfate group, a carboxyl group, or a sulfo group, and
the compound is dextran sulfate, polyacrylic acid, or polystyrene sulfonic acid.

3. The system for recovering an antigen in a solvent according to claim 1, wherein
the charged particles each contain 10 to 60 mol% of the cationic lipid, 6 to 14 mol% of the PEG-containing lipid, 20 to 50 mol% of the cholesterol, and 0 to 30 mol% of the neutral lipid, and
the average zeta potential of the charged particles is -15 to +10 mV.

4. The system for recovering an antigen in a solvent according to claim 1, wherein
the charged particles each contain 20 to 60 mol% of the cationic lipid, 8 to 14 mol% of the PEG-containing lipid, 20 to 50 mol% of the cholesterol, and 0 to 25 mol% of the neutral lipid, and
the average zeta potential of the charged particles is -10 to +5 mV.

5. The system for recovering an antigen in a solvent according to claim 1, wherein the charged particles have an average particle size of 200 nm or less.

6. The system for recovering an antigen in a solvent according to claim 1, wherein the charged particles have an average particle size of 100 nm or less.

7. The system for recovering an antigen in a solvent according to claim 1, wherein the adsorption carrier has an average zeta potential of -20 mV or less.

8. The system for recovering an antigen in a solvent according to claim 1, wherein the adsorption carrier has an average zeta potential of -30 mV or less.

9. The system for recovering an antigen in a solvent according to claim 1, wherein a concentration of the charged particles in the mixing unit is 0.01 to 2.0 mg/ml.

10. The system for recovering an antigen in a solvent according to claim 1, wherein
the mixing unit and the recovery unit are connected to each other by a first flow path,
the mixing unit is further connected to a second flow path,
the recovery unit is further connected to a third flow path,
the system for recovering an antigen in a solvent includes a pump configured to control a flow rate of the solvent, and
the pump is configured to adjust a flow rate of the solvent in the third flow path to 100 ml/min or less.

11. A method for recovering an antigen in a solvent, comprising:
a step of charging charged particles into a solvent and stirring the charged particles; and
a step of recovering the charged particles mixed with the solvent, wherein
the charged particles each contain 3 to 70 mol% of a cationic lipid, 1 to 20 mol% of a PEG-containing lipid, 20 to 50 mol% of cholesterol, and 0 to 65 mol% of a neutral lipid,
surfaces of the charged particles are modified with antibodies,
an average zeta potential of the charged particles is -20 to +15 mV, and
the charged particles mixed with the solvent are recovered by electrostatic adsorption via an adsorption carrier having a surface on which a compound having an anionic functional group is immobilized.
